# EUROPEAN PATENT SPECIFICATION

(11) **EP 4 231 926 B1**
(45) Date of publication and mention of the grant of the patent: **09.12.2026**
(21) Application number: 21810827.2
(22) Date of filing: 25.10.2021
(51) Int. Cl.: A61B 17/04

(54) **TISSUE REPAIR SYSTEMS**
GEWEBEREPARATURSYSTEME
SYSTÈMES DE RÉPARATION TISSULAIRE

(30) Priority: 26.10.2020 US 202063105646 P; 26.10.2020 US 202063105654 P
(43) Date of publication of application: 30.08.2023
(73) Proprietor: Smith & Nephew, Inc., Memphis, TN 38116 (US); Smith & Nephew Orthopaedics AG, 6300 Zug (CH); Smith & Nephew Asia Pacific Pte. Limited, Singapore 138565 (SG)
(72) Inventor: HOUSMAN, Mark E., Memphis, Tennessee 38116 (US)
(74) Representative: Appleyard Lees IP LLP
(86) International application number: PCT/US2021/056480
(87) International publication number: WO 2022/093706

(56) References cited:
- AU-A1- 2014 353 044
- US-A1- 2018 263 617
- US-A1- 2019 261 974
- US-B2- 8 277 484

## Description

### FIELD

The present disclosure relates to tissue repair systems and, more specifically, to tissue repair systems including anchors for attaching soft tissue to bone.

### BACKGROUND

Surgical procedures, such as rotor cuff repair, generally require reattachment of soft tissue to bone. To achieve this, surgeons often place anchors in the bone and pass sutures attached to the anchor through the tissue to securely retain the tissue in place. Some procedures require placement of multiple anchors to achieve the repair.

To reduce the amount of bone stock removed by the anchors and to minimize invasiveness, surgeons generally prefer using the smallest anchors possible. Smaller anchors also allow surgeons to place more anchors and provide more points of fixation, potentially enhancing patient healing. Smaller anchors, however, result in a problematic tradeoff between a reduced interior volume of the anchor and a weakened drive support structure. To maintain structural integrity during insertion, the drive elements of the anchor must withstand the torsion required for insertion of the anchor into bone. For example, the anchor may include drive ribs within an internal volume of the anchor to provide a structural element for a driver to apply torsion during insertion. However, in smaller anchors, drive ribs of adequate depth and size to support the anchor also limit the size and amount of suture attachable to the anchor. A need therefore exists for an anchor capable of withstanding torsional drive forces during anchor insertion and yet having a sufficiently small profile to avoid occlusion of internal suture passages. AU2014353044A1 relates to an anchor having a controlled driver orientation. US2018263617A1 relates to a device for fixing soft tissue to a bone tissue. US2019261974A1 relates to a fenestrated locking suture anchor assembly. US8277484B2 relates to suture anchors.

### SUMMARY

The invention is defined in the appended claims.

Tissue repair systems and methods of assembly described herein include suture anchors comprising a distal transfer eyelet. The use of transfer eyelet in various anchor configurations enables diameter reduction of the anchor, as well as enlargement of the driver cannulation. This in turn increases the amount and size of suture loadable into the system. The invention is defined in the appended claims.

Tissue repair systems of this disclosure include a driver having a shaft including a distal end defining two grooves. The shaft defines an internal cannulation extending through the shaft. A proximal portion of the cannulation has a diameter selected to be larger than a diameter of a distal portion of the cannulation. An anchor is engageable with the distal end of the shaft of the driver. The anchor has an anchor body including at least one open helical coil defining an internal volume communicating with a region exterior to the at least one open helical coil through a spacing between turns of the at least one open helical coil. The internal volume is configured for insertion of the distal end of the shaft of the driver. Two ribs are disposed within the internal volume. The ribs are configured to engage the grooves of the shaft of the driver. A flexible member extends through an opening at a distal end of the anchor body and forms a loop within the interior volume. The loop is configured to be disposed in the proximal portion of the cannulation of the shaft. The opening at the distal end of the anchor body is defined by a support member. At least one of the ribs is a truncated rib that extends proximally from the support member to a region distal to a proximal end of the anchor.

A reading of the following detailed description and a review of the associated drawings will make apparent the advantages of these and other features. Both the foregoing general description and the following detailed description serve as an explanation only and do not restrict aspects of the disclosure as claimed.

### BRIEF DESCRIPTION OF THE DRAWINGS

Reference to the detailed description, combined with the following figures, will make the disclosure more fully understood, wherein:
FIG. 1 shows an example of the tissue repair system of this disclosure, including an anchor, a delivery device, and a transfer eyelet, in an assembled view;
FIG. 2 shows the distal end of a shaft of the delivery device of FIG. 1;
FIGS. 3A and 3B show cross-sectional views of the tissue repair system of FIG. 1 with the shaft inserted (FIG. 3A) and the shaft removed (FIG. 3B);
FIGS. 4A-C show cross-sectional views of the tissue repair system of FIG. 1 at a proximal region of the system (FIG. 4A), a central region of the system (FIG. 4B) and a distal region of the system (FIG. 4C);
FIGS. 5A and 5B show a distal face of the tissue repair system of FIG. 1 with the transfer eyelet removed (FIG. 5A) and the transfer eyelet inserted (FIG. 5B);
FIGS. 6A and 6B illustrate a method of loading the transfer eyelet and repair suture into the anchor of FIG. 1;
FIGS. 7A-E illustrate a method of loading the repair suture into the delivery device of FIG. 1;
FIG. 8 shows another example of the tissue repair system of this disclosure, including an anchor, a delivery device, and a transfer eyelet, in an assembled view;
FIG. 9 shows the distal end of a shaft of the delivery device of FIG. 8;
FIGS. 10A and 10B show cross-sectional views of the tissue repair system of FIG. 8 with the shaft inserted (FIG. 10A) and the shaft removed (FIG. 10B); and
FIGS. 11A-C show cross-sectional views of the tissue repair system of FIG. 8 at a proximal region of the system (FIG. 11A), a central region of the system (FIG. 11B) and a distal region of the system (FIG. 11C).

### DETAILED DESCRIPTION

In the following description, like components have the same reference numerals, regardless of different illustrated examples. To illustrate examples clearly and concisely, the drawings may not necessarily reflect appropriate scale and may have certain features shown in somewhat schematic form. The disclosure may describe and/or illustrate features in one example, and in the same way or in a similar way in one or more other examples, and/or combined with or instead of the features of the other examples.

In the specification and claims, for the purposes of describing and defining the invention, the terms "about" and "substantially" represent the inherent degree of uncertainty attributed to any quantitative comparison, value, measurement, or other representation. The terms "about" and "substantially" moreover represent the degree by which a quantitative representation may vary from a stated reference without resulting in a change in the basic function of the subject matter at issue. Open-ended terms, such as "comprise," "include," and/or plural forms of each, include the listed parts and can include additional parts not listed, while terms such as "and/or" include one or more of the listed parts and combinations of the listed parts.

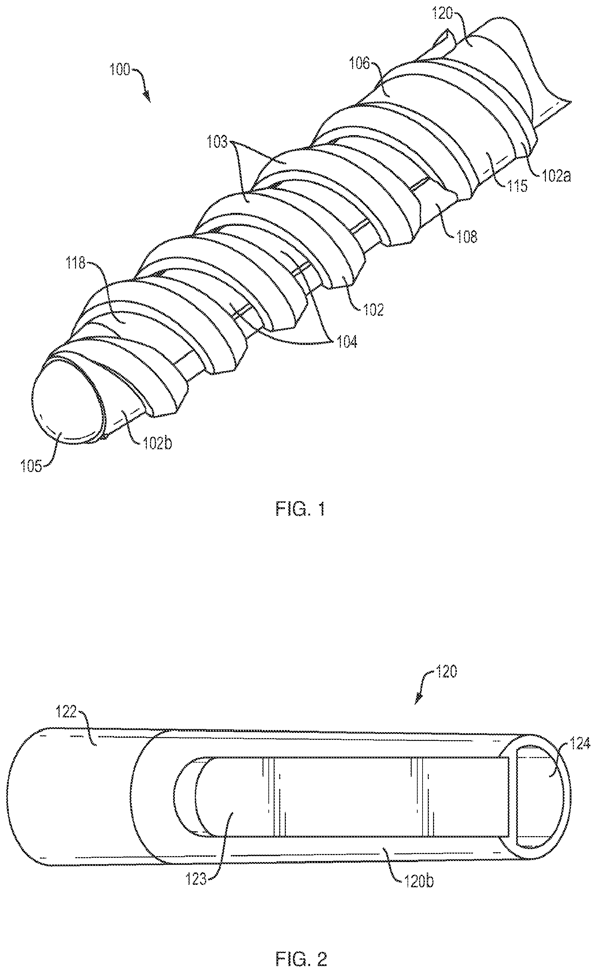

FIG. 1 shows an example of a tissue repair system 100 of this disclosure in an assembled view. The system 100 comprises an anchor 102 having a body 106 including a proximal end 102a and a distal end 102b. Most of the anchor 102 includes a screw thread 103 in the form of an open helical coil - i.e., a connected series of continuous regularly spaced turns of the thread 103 extending in a helical or spiral form substantially from the proximal end 102a to the distal end 102b with apertures 104 defined by the space between the turns of the thread 103. The body 106 of the anchor 102 defines an internal volume communicating with a region exterior to the body 106 through the apertures 104. In examples, webbing 115 may extend between any two adjacent turns of the thread 103, although the disclosure also contemplates anchors 102 without webbing. The distal end 102b also includes a transfer eyelet 105 extending through a support member 118 into the interior volume of the anchor 102. Additionally, a longitudinally extending rib 108 extends from the support member 118 along an interior surface of the thread 103, contacting at least two adjacent turns of the thread 103. In the example of FIG. 1, only a single rib 108 extends through the anchor 102. When the anchor 102 couples to the delivery device 120, the rib 108 engages with a groove of the delivery device 120, as further described below. In examples, the anchor 102 may comprise polymers (e.g., polyetheretherketone) (PEEK)), bioabsorbable materials, metals (e.g., surgical steel, titanium), or any other suitable material.

FIG. 2 shows an example of a distal end 120b of a shaft 122 of the delivery device 120. The distal end 120b defines a groove 123 on an outer surface of the shaft 122. When inserted into the anchor 102 of FIG. 1, the rib 108 of the anchor 102 houses within the groove 123. The shaft 122 further defines a cannulation 124 such that when the anchor 102 engages the shaft 122, repair sutures attached to the anchor 102 extend through the cannulation 124.

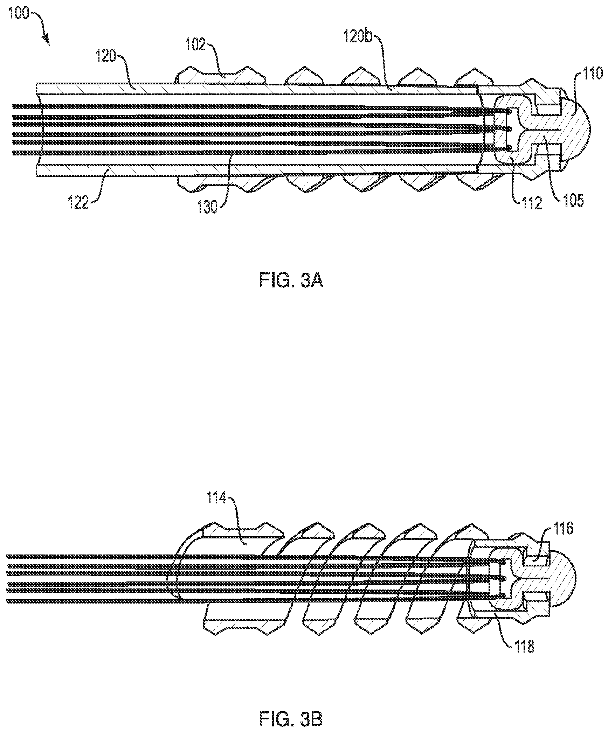

FIGS. 3A and 3B show examples the tissue repair system 100 in cross-sectional views, both with the shaft 122 inserted into the anchor 102 (FIG. 3A), and with the shaft 122 removed from the anchor 102 (FIG. 3B). In examples, the transfer eyelet 105 may comprise a flexible member, such as a suture, having a knot 110 and a loop 112 extending proximally from the knot 110 into the internal volume 114 of the anchor 102. More particularly, the loop 112 extends through a distal opening 116 defined by the support member 118. A size of the opening 116 is selected such that the knot 110 cannot pass through opening 116 into the internal volume 114. One or more repair sutures 130 couple to the loop 112 and extend through the cannulation 124 of the delivery device 120. As shown, the loop 112 remains distal to the distal end 120b of the shaft 122 as the distal end 120b abuts a proximal end of the support member 118.

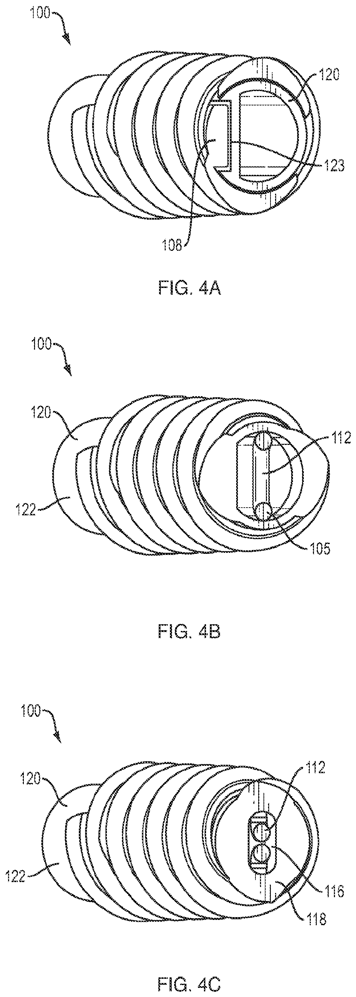

FIGS. 4A-C show additional cross-sectional views of the tissue repair system 100. FIG. 4A shows a cross-sectional view through a more proximal portion of the system 100, including the rib 108 engaged with the groove 123 of the delivery device 120. FIG. 4B shows a cross-sectional view through a more central portion of the system 100, including the entire loop 112 of the transfer eyelet 105 distal to the shaft 122 of the delivery device 120. FIG. 4C shows a cross-sectional view through a more distal portion of the system 100, including the limbs of the loop 112 passing through the opening 116 in the support member 118.

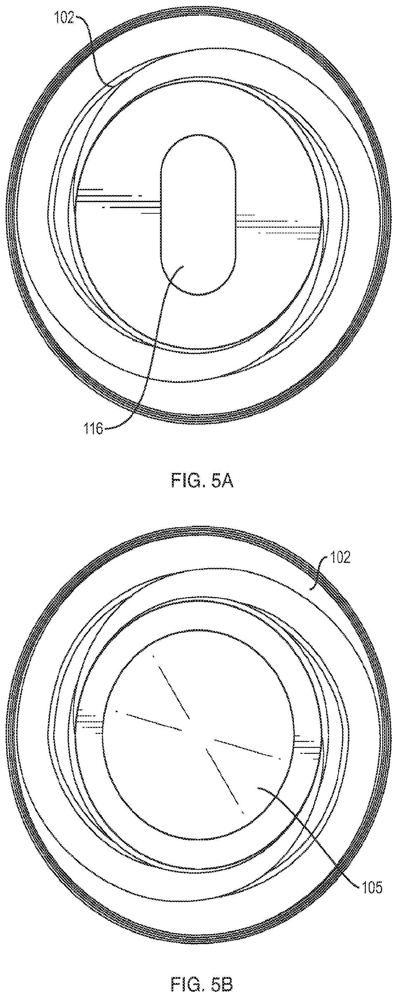

FIGS. 5A and 5B show a distal face of the anchor 102, with the transfer eyelet 105 removed from the opening 116 (FIG. 5A) and the transfer eyelet 105 inserted into the opening 116 (FIG. 5B). In examples, a shape of the opening 116 substantially defines an oval. This oval shape advantageously allows preferential orientation of the repair sutures within the anchor 102 such that the repair sutures align with a non-circular cannulation 124 of the delivery device 120. However, the disclosure also contemplates circular and other convenient shapes of the opening 116.

Advantageously, the use of the distal transfer eyelet 105 and the single rib 108 enable a diameter reduction of the anchor 102 compared to anchors having more than one internal rib. In examples, the anchor 102 can have an outer diameter as small as 3.5 mm. The design also allows maximization of the amount and/or size of the repair suture 130 loadable into the anchor 102. Additionally, the repair sutures 130 need not be folded over when inserted into the anchor 102 through the cannulation 124 of the delivery device 120, as in current tissue repair systems, allowing easier loading of the repair suture 130. The small diameter of the anchor 102 relative to a size of the single rib 108 also allows the anchor 102 to maintain structural integrity during insertion.

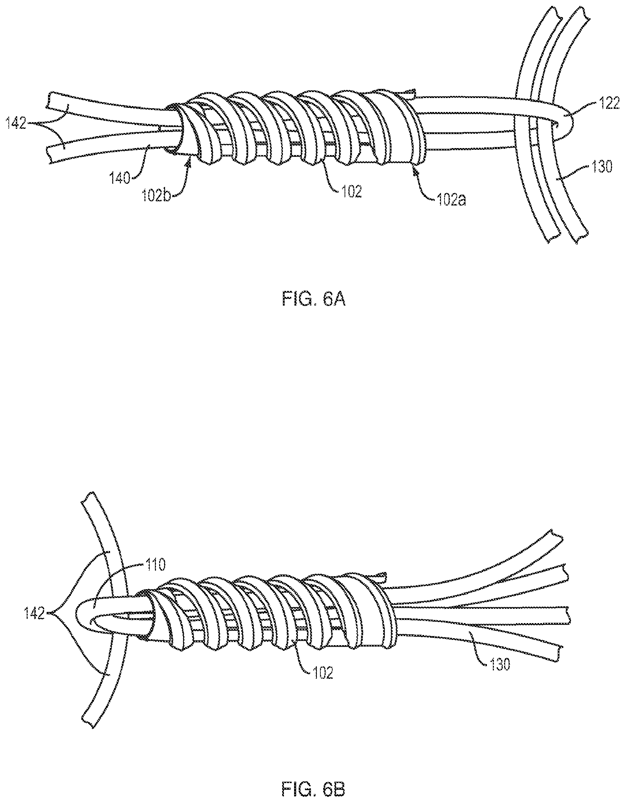

FIGS. 6A and 6B illustrate a method of loading the anchor 102 with repair suture 130 for use in a tissue repair. As shown in FIG. 6A, to form the transfer eyelet 105, a folded-over flexible member 140, such as a #2 suture, is passed through the anchor 102 such that the loop 122 extends from the proximal end 102a and the suture limbs 142 extend from the distal end 102b. Repair sutures 130, which may include sutures, tapes or combinations thereof, are coupled to the loop 122, such as by passing through the looped portion 122. As shown in FIG. 6B, the suture limbs 142 are pulled in a distal direction to draw the loop 122 with the repair sutures 130 into the anchor 102. The knot 110 is tied with the suture limbs 142 and the suture limbs 142 are trimmed (not shown) from the knot 110 before the repair sutures 130 are loaded into the delivery device 120.

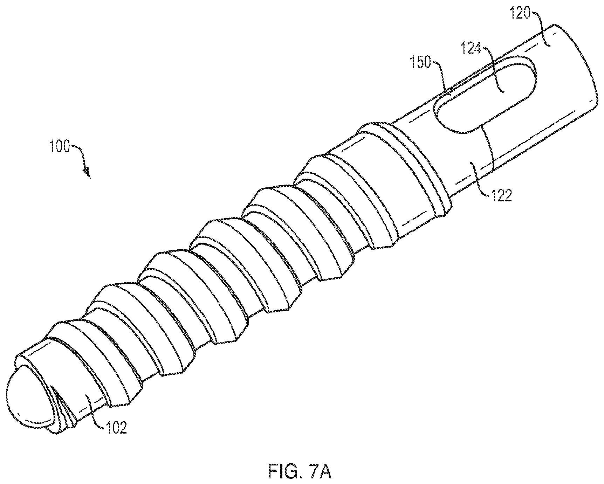

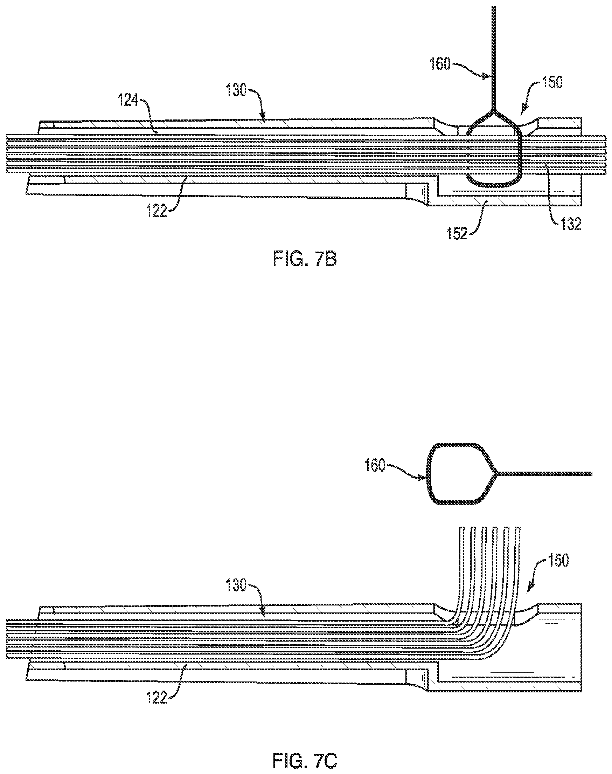

FIGS. 7A-7E illustrate a method of loading the repair sutures 130 into the delivery device 120. FIG. 7A shows the tissue repair system 100 of FIG. 1 rotated to show an aperture 150 defined in an outer surface of the shaft 122 in a region just proximal to the anchor 102. The aperture 150 communicates with the cannulation 124 of the shaft 122. While FIG. 7A shows the aperture 150 having an oval shape, the disclosure contemplates other convenient shapes of the aperture 150. FIG. 7B shows that a diameter of the cannulation 124 can increase proximally from a region 152 of the aperture 150 because the shaft 122 need no longer engage the internal volume of the anchor 102. Before inserting the shaft 122 into the anchor 102, a suture puller 160 or other convenient instrument is inserted into the aperture 150. The repair sutures 130 are loaded into a distal end of the cannulation 124 up to the aperture 150, for example, by hand. To aid the insertion, the repair suture 130 may comprise heat-stiffened ends 132 that allow pushing of the ends 132 into the cannulation 124 and receipt through a loop of the suture puller 160. Preferably, a length of the heat-stiffened ends 132 is at least 20 mm. FIG. 7C shows the suture puller 160 used to pull the repair sutures 130 through the aperture 150 such that the repair sutures 130 are temporarily parked outside of the shaft 122. Although FIG. 7C shows all of the repair sutures 130 parked outside of the shaft 122, only a subset of the repair sutures 130 need to extend through the aperture 150 to fully load the system 100. The disclosure also contemplates that other means, such as a removeable ramp feature, could divert the heat-stiffened ends 132 through the aperture 150. The shaft 122 can then be inserted into the anchor 102.

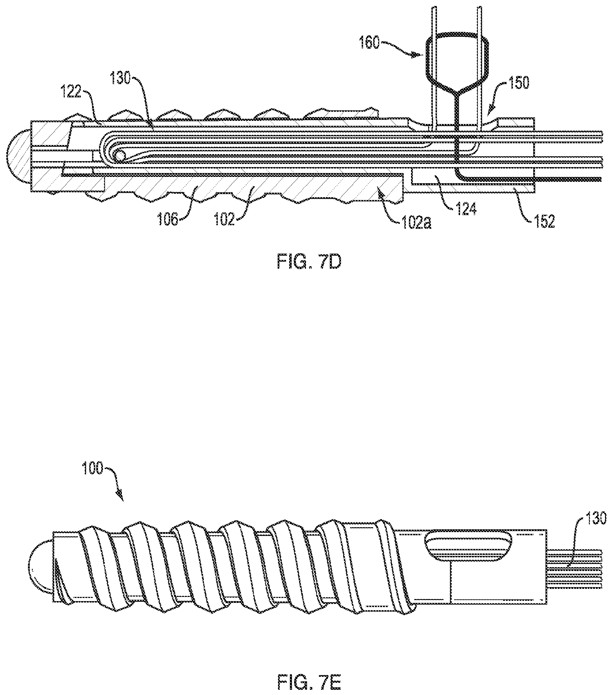

FIG. 7D shows the shaft 122 inserted into the anchor 102 such that the region 152 of the aperture 150 abuts the proximal end 102a of the anchor body 106. The repair sutures 130 can now be passed through the remainder of the cannulation 124 by passing the suture puller 160 or other convenient tool through a proximal end of the cannulation 124 to retrieve the repair sutures 130 back into the cannulation 124, as shown in FIG. 7E. After this step, all repair sutures 130 are fully loaded into the tissue repair system 100, making the tissue repair system 100 ready for use.

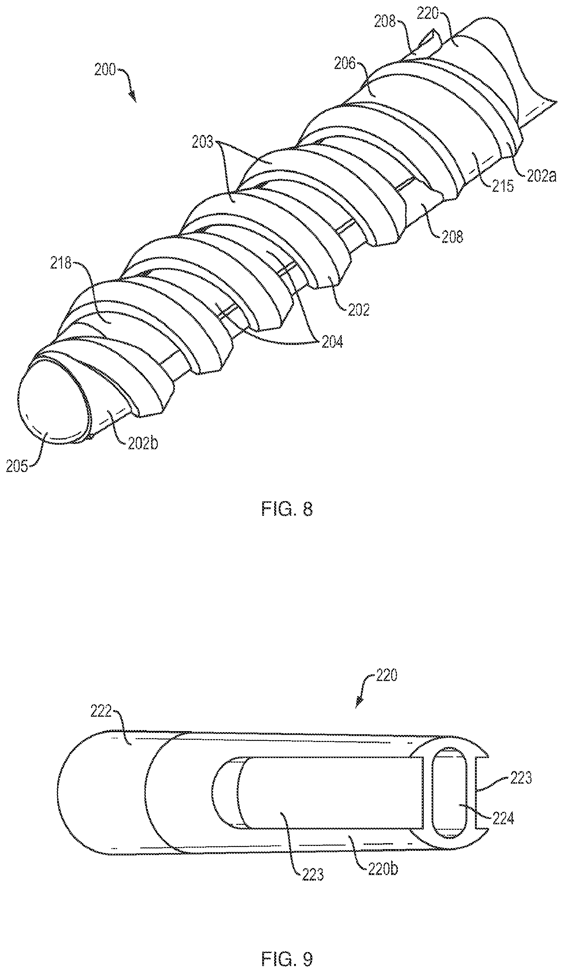

FIG. 8 shows another example of a tissue repair system 200 of this disclosure in an assembled view. Tissue repair system 200 is substantially similar to the tissue repair system 100, except as described below. The tissue repair system 200 comprises an anchor 202 having a body 206 including a proximal end 202a and a distal end 202b. Most of the anchor 202 includes a screw thread 203 in the form of an open helical coil - i.e., a connected series of continuous regularly spaced turns of the thread 203 extending in a helical or spiral form substantially from the proximal end 202a to the distal end 202b with apertures 204 defined by the space between the turns of the thread 203. The body 206 of the anchor 202 defines an internal volume communicating with a region exterior to the body 206 through the apertures 204. In examples, webbing 215 may extend between any two adjacent turns of the thread 203, although the disclosure also contemplates anchors 202 without webbing. The distal end 202b also includes a transfer eyelet 205 extending through a support member 218 into the interior volume of the anchor 202. Additionally, a plurality of longitudinally extending ribs 208 extends from the support member 218 along an interior surface of the thread 203, contacting at least two adjacent turns of the thread 203. In the example of FIG. 8, two diametrically opposed ribs 208 extend through the anchor 202. When the anchor 202 couples to the delivery device 220, the ribs 208 engage with a plurality of grooves of the delivery device 220, as further described below. In examples, the anchor 202 may comprise polymers, bioabsorbable materials, metals, or any other suitable material.

FIG. 9 shows an example of a distal end 220b of a shaft 222 of the delivery device 220. The distal end 220b defines the plurality grooves 223 on an outer surface of the shaft 222. In the example of FIG. 9, the shaft 222 defines two diametrically opposed grooves 223. When inserted into the anchor 202 of FIG. 8, the ribs 208 of the anchor 202 house within the grooves 223. The delivery device 220 defines a cannulation 224 such that when the anchor 202 engages the shaft 222, both the transfer eyelet 205 and repair sutures attached to the transfer eyelet 205 extend through the cannulation 224.

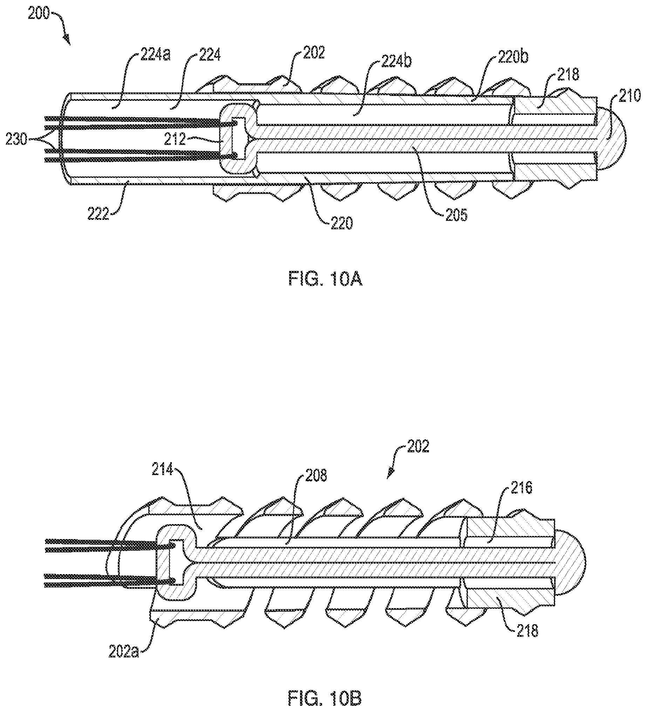

FIGS. 10A and 10B show examples the tissue repair system 200 in cross-sectional views, both with the shaft 222 inserted into the anchor 202 (FIG. 10A), and with the shaft 222 removed from the anchor 202 (FIG. 10B). In examples, the transfer eyelet 205 may comprise a flexible member, such as a suture, having a knot 210 and a loop 212 extending proximally from the knot 210 into the internal volume 214 of the anchor 202. More particularly, the loop 212 extends through a distal opening 216 defined by the support member 218. A size of the opening 216 is selected such that the knot 210 cannot pass through opening 216 into the internal volume 214 of the anchor 202. One or more repair sutures 230 couple to the loop 212 and extend through the cannulation 224 of the delivery device 220. As shown in FIG. 10B, rather than extending an entire length of the anchor 202, the truncated ribs 208 extend proximally from the support member 218 to a region distal to the proximal end 202a of the anchor 202. As such, a proximal portion 224a of the cannulation 224 can have a diameter selected to be wider than a diameter of a distal portion 224b of the cannulation 224 in the region of the shaft 222 where the grooves 223 do not engage the ribs 208. The limbs of the loop 212 extend through the cannulation 224 such that the loop 212 resides in the proximal portion 224a of the cannulation 224 as the distal end 220b of the shaft 222 abuts a proximal end of the support member 218. The smaller diameter of the distal portion 224b further prevents the loop 212 from entering the distal portion 224b. While FIG. 10B illustrates one of two truncated ribs 208, the disclosure also contemplates one fully-extending rib 208 and one truncated rib 208 extending through the anchor 202.

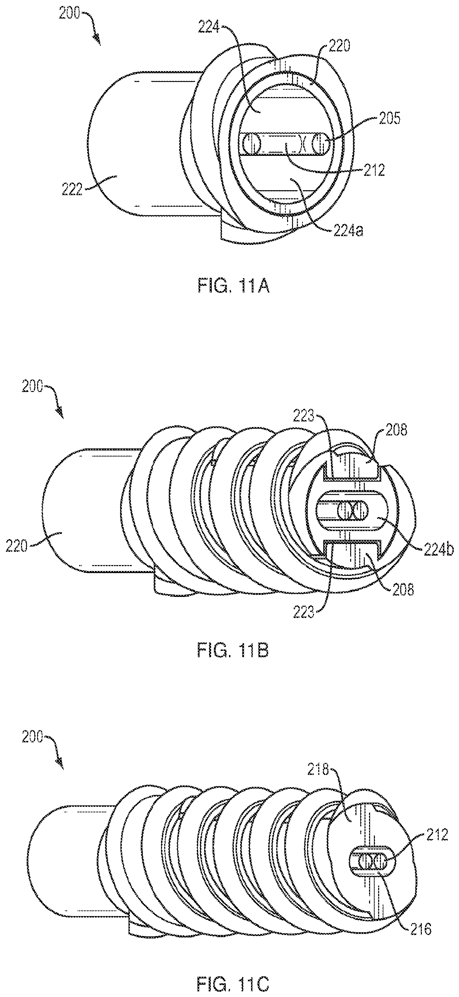

FIGS. 11A-C show additional cross-sectional views of the tissue repair system 200. FIG. 11A shows a cross-sectional view through a more proximal portion of the system 200, including the entire loop 212 of the transfer eyelet 205 in the proximal portion 224a of the cannulation 224. FIG. 11B shows a cross-sectional view through a more central portion of the system 200, including the ribs 208 engaged with the grooves 223 of the delivery device 220. FIG. 11C shows a cross-sectional view through a more distal portion of the system 200, including the limbs of the loop 212 passing through the opening 216 in the support member 218. Like in the tissue repair system 100, the oval shape of the opening 216 advantageously allows preferential orientation of the repair sutures within the anchor 202 such that the repair sutures align with a non-circular cannulation 224 of the distal portion 224b.

Advantageously, the use of the transfer eyelet 205 and the truncated ribs 208 enable a diameter reduction of the anchor 202 compared to anchors having ribs extending an entire length of the anchor. In examples, the anchor 102 can have an outer diameter as small as 3.5 mm. The design also allows maximization of the amount and/or size of the repair suture 230 loadable into the anchor 202. Additionally, the repair sutures 230 need not be folded when inserted into the anchor 202 through the cannulation 224 of the delivery device 220, as in current tissue repair systems, allowing easier loading of repair suture 230 into the tissue repair system 200. The repair suture 230 may be loaded into the tissue repair system 200 in a similar manner as described with regard to FIGS. 6A-6C and 7A-7E.

## Claims

1. A tissue repair system (200) comprising:
a driver (220) comprising a shaft (222) including a distal end (220b) defining two grooves (223), the shaft (222) defining an internal cannulation (224) extending through the shaft (222); and
an anchor (202) engageable with the distal end (220b) of the shaft (222) of the driver (220), the anchor (202) comprising:
an anchor body (206) including at least one open helical coil defining an internal volume communicating with a region exterior to the at least one open helical coil through a spacing (204) between turns of the at least one open helical coil, the internal volume configured for insertion of the distal end (220b) of the shaft (222) of the driver (220); and
two ribs (208) disposed within the internal volume, the ribs (208) configured to engage the grooves (223) of the shaft (222) of the driver (220); **characterized in that** a proximal portion (224a) of the cannulation (224) has a diameter selected to be larger than a diameter of a distal portion (224b) of the cannulation (224), and
the anchor further comprises a flexible member extending through an opening (216) at a distal end (202b) of the anchor body (206) and forming a loop (212) within the interior volume, the loop (212) configured to be disposed in the proximal portion (224b) of the cannulation (224) of the shaft (222),
wherein the opening (216) at the distal end (202b) of the anchor body (206) is defined by a support member (218), wherein the ribs (208) are truncated ribs that extend proximally from the support member (218) to a region distal to the proximal end (202a) of the anchor (202).

2. The tissue repair system (200) of claim 1, wherein the ribs (208) are connected to at least two turns of at the least one open helical coil.

3. The tissue repair system (200) of claim 1, wherein the loop (212) is further configured to be coupled to at least one suture (230) extending through the interior volume of the anchor body (206).

4. The tissue repair system (200) of claim 1, wherein the proximal portion (224a) of the cannulation (224) is configured for passage of the at least one suture (230) coupled to the loop (212).

5. The tissue repair system (200) of claim 1, wherein an outer surface of the shaft (222) defines an aperture in communication with the cannulation (224) such that at least one of the at least one suture (230) is routable through the aperture.

## Patentansprüche

1. Gewebereparatursystem (200), umfassend:
einen Treiber (220), der einen Schaft (222) umfasst, umfassend ein distales Ende (220b), das zwei Nuten (223) definiert, wobei der Schaft (222) eine interne Kanülierung (224) definiert, die sich durch den Schaft (222) erstreckt; und
einen Anker (202), der mit dem distalen Ende (220b) des Schafts (222) des Treibers (220) in Eingriff gebracht werden kann, wobei der Anker (202) Folgendes umfasst:
einen Ankerkörper (206), der mindestens eine offene spiralförmige Wicklung beinhaltet, die ein Innenvolumen definiert, das mit einem Bereich außerhalb der mindestens einen offenen spiralförmigen Wicklung durch einen Abstand (204) zwischen Windungen der mindestens einen offenen spiralförmigen Wicklung kommuniziert, wobei das Innenvolumen zum Einführen des distalen Endes (220b) des Schafts (222) des Treibers (220) konfiguriert ist; und zwei Rippen (208), die innerhalb des Innenvolumens angeordnet sind, wobei die Rippen (208) dazu konfiguriert sind, in die Nuten (223) des Schafts (222) des Treibers (220) einzugreifen;
**dadurch gekennzeichnet, dass** ein proximaler Abschnitt (224a) der Kanülierung (224) einen Durchmesser aufweist, der so gewählt ist, dass er größer als ein Durchmesser eines distalen Abschnitts (224b) der Kanülierung (224) ist, und
der Anker ferner ein flexibles Element umfasst, das sich durch eine Öffnung (216) an einem distalen Ende (202b) des Ankerkörpers (206) erstreckt und eine Schlaufe (212) innerhalb des Innenvolumens bildet, wobei die Schlaufe (212) dazu konfiguriert ist, im proximalen Abschnitt (224b) der Kanülierung (224) des Schafts (222) angeordnet zu werden,
wobei die Öffnung (216) am distalen Ende (202b) des Ankerkörpers (206) durch ein Stützelement (218) definiert ist, wobei die Rippen (208) abgestumpfte Rippen sind, die sich proximal vom Stützelement (218) zu einem Bereich distal zum proximalen Ende (202a) des Ankers (202) erstrecken.

2. Gewebereparatursystem (200) nach Anspruch 1, wobei die Rippen (208) mit mindestens zwei Windungen der mindestens einen offenen spiralförmigen Wicklung verbunden sind.

3. Gewebereparatursystem (200) nach Anspruch 1, wobei die Schlaufe (212) ferner dazu konfiguriert ist, mit mindestens einem Nahtmaterial (230) gekoppelt zu werden, das sich durch das Innenvolumen des Ankerkörpers (206) erstreckt.

4. Gewebereparatursystem (200) nach Anspruch 1, wobei der proximale Abschnitt (224a) der Kanülierung (224) für den Durchgang des mindestens einen Nahtmaterials (230) konfiguriert ist, das mit der Schlaufe (212) gekoppelt ist.

5. Gewebereparatursystem (200) nach Anspruch 1, wobei eine Außenfläche des Schafts (222) eine Öffnung definiert, die mit der Kanülierung (224) in Verbindung steht, so dass mindestens eines des mindestens einen Nahtmaterials (230) durch die Öffnung leitbar ist.

## Revendications

1. Système de réparation tissulaire (200) comprenant :
un dispositif d'entraînement (220) comprenant une tige (222) comprenant une extrémité distale (220b) définissant deux rainures (223), la tige (222) définissant une canulation interne (224) s'étendant à travers la tige (222) ; et
un ancrage (202) apte à coopérer avec l'extrémité distale (220b) de la tige (222) du dispositif d'entraînement (220), l'ancrage (202) comprenant :
un corps d'ancrage (206) comprenant au moins un enroulement hélicoïdal ouvert définissant un volume interne communiquant avec une région extérieure à l'au moins un enroulement hélicoïdal ouvert à travers un espacement (204) entre des spires de l'au moins un enroulement hélicoïdal ouvert, le volume interne étant configuré pour l'insertion de l'extrémité distale (220b) de la tige (222) du dispositif d'entraînement (220) ; et deux nervures (208) disposées à l'intérieur du volume interne, les nervures (208) étant configurées pour engager les rainures (223) de la tige (222) du dispositif d'entraînement (220) ;
**caractérisé en ce qu'**une partie proximale (224a) de la canulation (224) présente un diamètre choisi pour être plus grand qu'un diamètre d'une partie distale (224b) de la canulation (224) et
l'ancrage comprend en outre un élément flexible s'étendant à travers une ouverture (216) au niveau d'une extrémité distale (202b) du corps d'ancrage (206) et formant une boucle (212) à l'intérieur du volume intérieur, la boucle (212) étant configurée pour être disposée dans la partie proximale (224b) de la canulation (224) de la tige (222),
l'ouverture (216) au niveau de l'extrémité distale (202b) du corps d'ancrage (206) étant définie par un élément de support (218), les nervures (208) étant des nervures tronquées qui s'étendent de manière proximale à partir de l'élément de support (218) jusqu'à une région distale à l'extrémité proximale (202a) de l'ancrage (202).

2. Système de réparation tissulaire (200) selon la revendication 1, les nervures (208) étant connectées à au moins deux spires de l'au moins un enroulement hélicoïdal ouvert.

3. Système de réparation tissulaire (200) selon la revendication 1, la boucle (212) étant en outre configurée pour être couplée à au moins une suture (230) s'étendant à travers le volume intérieur du corps d'ancrage (206).

4. Système de réparation tissulaire (200) selon la revendication 1, la partie proximale (224a) de la canulation (224) étant configurée pour le passage de l'au moins une suture (230) couplée à la boucle (212).

5. Système de réparation tissulaire (200) selon la revendication 1, une surface extérieure de la tige (222) définissant une ouverture en communication avec la canulation (224) de telle sorte qu'au moins l'une de l'au moins une suture (230) peut être acheminée à travers l'ouverture.
